# EUROPÄISCHE PATENTSCHRIFT

(11) **EP 2 170 301 B1**
(45) Veröffentlichungstag und Bekanntmachung des Hinweises auf die Patenterteilung: **03.09.2014**
(21) Anmeldenummer: 08785157.2
(22) Anmeldetag: 28.07.2008
(51) Int. Cl.: A61K 9/51

(54) **VIRUS-MODIFIZIERTE BAKTERIENGHOSTS**
VIRUS-MODIFIED BACTERIA GHOSTS
FANTÔMES BACTÉRIENS MODIFIÉS PAR VIRUS

(30) Priorität: 27.07.2007 EP 07014799
(43) Veröffentlichungstag der Anmeldung: 07.04.2010
(73) Patentinhaber: Lubitz, Werner, 3420 Klosterneuburg/Kritzendorf (AT)
(72) Erfinder: Lubitz, Werner, 3420 Klosterneuburg/Kritzendorf (AT)
(74) Vertreter: Weiss, Wolfgang
(86) Internationale Anmeldenummer: PCT/EP2008/006207
(87) Internationale Veröffentlichungsnummer: WO 2009/015852

(56) Entgegenhaltungen:
- WO-A-00/53163
- DE-A1- 19 903 345
- MAYR U B ET AL: "Bacterial ghosts as antigen delivery vehicles" ADVANCED DRUG DELIVERY REVIEWS, ELSEVIER BV, AMSTERDAM, NL, Bd. 57, Nr. 9, 17. Juni 2005 (2005-06-17), Seiten 1381-1391, XP004921417 ISSN: 0169-409X

## Beschreibung

Die Erfindung betrifft Virus-modifizierte Bakterien Ghosts (BGs) und deren Verwendung, z.B. als Impfstoffpartikel, Träger- und Targetingvehikel für Wirkstoffe sowie für Anwendungen in der Nanotechnologie. Bakterienviren werden auch als Bakteriophagen (Bph) oder Phagen bezeichnet.

Leere Bakterienhüllen, so genannte BGs, können durch kontrollierte, heterologe Expression eines Gens, das eine partielle Lyse der Zellmembran bewirkt, in gram-negativen Bakterien hergestellt werden (EP-A-0 291 021). Ein Beispiel für ein derartiges lytisches Gen ist das Gen E des Bakteriophagen PhiX174, das für ein Polypeptid kodiert, welches in den Zellwandkomplex gram-negativer Bakterien inseriert und durch die Oligomerisierung zur Ausbildung einer transmembranen Tunnelstruktur durch die innere und äußere Membran führt. Der innere Durchmesser dieser Tunnelstruktur kann je nach Lysebedingungen 40 bis 200 nm oder 500 bis 1000 nm betragen. Durch diesen Tunnel wird das cytoplasmatische Material der Zelle freigesetzt und eine leere Zellhülle mit intakter Morphologie zurückgelassen. Die Verwendung von BGs als Totimpfstoffe oder Adjuvanzien sowie die Herstellung rekombinanter BGs, die in ihrer Membran heterologe Oberflächenproteine tragen, wird in WO 91/13555 und WO 93/01791 beschrieben.

Weiterhin sind BGs, wie in WO 00/053163 beschrieben hervorragend als Träger oder Targetingvehikel für Wirkstoffe geeignet. Ein erster Vorteil der BGs besteht darin, dass die Applikation ohne Weiteres über den natürlichen Infektionsweg von Pathogenen oder durch natürliche Rezeptorerkennung von nicht-pathogenen Bakterien wie etwa über den Respirations- oder den Gastrointestinaltrakt oder andere Schleimhäute möglich ist. Dies gilt auch für Rezeptoren auf Pflanzen oder die Adsorption von Bakterien an nicht-belebte Materialien. Darüber hinaus bietet das Applikationssystem von Wirkstoffen über BGs als Träger aufgrund der Spezifität der BGs für verschiedene Gewebearten ein wirkungsvolles Targeting. Damit wird der Wirkstoff an den gewünschten Zielort, z.B. den entsprechenden potenziellen Infektionsort der Ausgangsbakterien, oder allgemein am gewünschten Zielort mit hoher Effizienz gebracht. Dieser Vorteil von natürlichen Bakterienhüllen als Vektoren kann bei anderen Applikationsformen, wie z.B. Liposomen, selbst mit eingebauten Proteinen oder Liganden nur mühsam und unzulänglich erreicht werden.

Da BGs herstellbar sind, die ausschließlich den gewünschten Wirkstoff enthalten, kann ein hoher Beladungsgrad und somit eine hohe Effizienz des Wirkstoffs erreicht werden. Zudem stellen BGs ein sicheres Trägermaterial dar, da es sich nicht um lebensfähige Organismen handelt. Schließlich handelt es sich bei BGs um Produkte mit einer hohen immunstimulatorischen Wirkung aufgrund des Vorhandenseins von Lipopolysacchariden und Peptidoglykanen, so dass keine zusätzlichen Adjuvanzien zugegeben werden müssen, da die BGs den Adjuvanzeffekt per se erfüllen.

In DE 199 03 345 werden Wirtszellen beschrieben, die sich zur Herstellung von BGs eignen, und die mindestens zwei funktionelle rekombinante Polypeptide umfassen. Von diesen Polypeptiden liegt mindestens eines in trägergebundener Form vor, wie z. B. in Form von Fusionspolypeptiden und/oder als Bestandteil rekombinanter Phagenstrukturen. Die trägergebundenen Polypeptide können Bestandteil der rekombinanten Phagenstrukturen, z. B. φX174 oder φCH1, sein. Ein Verfahren zur Herstellung (Bakteriophagen-) Bph-modifizierter Bakterienghosts durch Bph-Infektionen, insbesondere nach der bakteriellen Lyse wird nicht dargestellt.

Überraschenderweise wurde gefunden, dass BGs mit Bakterien-spezifischen Viren, sogenannten Bakteriophagen (Bph) infiziert werden können und dass die resultierenden Bph-modifizierten BGs für molekularbiologische und medizinische Anwendungen geeignet sind. Durch die Bph-infektion können physikalische und chemische Eigenschaften der BGs modifiziert werden. Weiterhin können auf diese Weise Targeting-Moleküle und Wirkstoffe in die BGs eingeschleust und/oder gebunden werden.

Der Gegenstand der Erfindung wird in den Ansprüchen festgelegt.

Ein Gegenstand der Erfindung ist somit ein Verfahren zur Herstellung von Bakteriophagen (Bph)-modifizierten Bakterienghosts (BG) durch Bph-Infektion von Bakterien nach der bakteriellen Lyse, umfassend (a) das Herstellen von Bakterienghosts und (b) das Inkubieren der Bakterienghosts mit Bph unter geeigneten Bedingungen, sodass eine Adsorption der Bph an der BG-Oberfläche stattfindet.

Ein weiterer Gegenstand der Erfindung sind Bakteriophagen (Bph)-modifizierte Bakterienghosts, die durch das erfindungsgemäße Verfahren erhältlich sind.

Bph modifizierten BGs können in der Molekularbiologie und in der medizinischen Diagnostik und Therapeutik als Träger- oder/und Targetingvehikel für einen Wirkstoff, z.B. als Vakzin oder Adjuvanz, als Nukleinsäurevektor oder für kombinatorische Anwendungen verwendet werden.

Ein weiterer Gegenstand der Erfindung umfasst daher die Verwendung der erfindungsgemäßen Bakteriophagen-modifizierten Bakterienghosts für Bakteriophagen (Bph)-Display und als konstruktive Elemente in der Nanotechnologie sowie Verfahren zur Herstellung einer pharmazeutischen Zusammensetzung, umfassend das Bereitstellen der erfindungsgemäßen BGs und das Inkontaktbringen der BGs mit einem Wirkstoff unter Bedingungen, die zu einer Verpackung des Wirkstoffs in den BGs führen.

Bph-infizierte BGs sind erhältlich durch Bph-infektion von entsprechenden Bakterien vor oder/und nach der E-induzierten Lyse. Die Bph-infektion erfolgt nach der bakteriellen Lyse.

Zur Infektion der BGs können beliebige Phagen verwendet werden, vorausgesetzt dass sie in der Lage sind, eine gegebene Bakterienzelle zu infizieren. Spezifische Beispiele für solche Bph sind Bph der Familie Styloviridae, wie etwa lambdoide Phagen, z.B. der für E.coli spezifische Bph Lambda und Mu-Phagen, Bph der Familie Myoviridae, wie etwa geradzahlige T-Phagen, z.B. T2, T4 und T6, Bph der Familie Podoviridae, wie etwa ungeradzahlige T-Phagen, z.B. T1, T3, T5 und T7, Bph der Familie Tectiviridae, z.B. pRD1, Bph der Familie Corticoviridae, z.B. pM2, Bph der Familie Plasmaviridae, Bph der Familie Mikroviridae, z.B. PhiX174, fadenförmige Bph der Familie Inoviridae, z.B. fd und M13, bei denen es sich um DNA-Bph handelt. Weiterhin geeignet sind auch RNA-Bph, z.B. Bph der Familie Leviviridae, z.B. MS2, F2 und Qβ. Die Infektion der BGs kann mit einer oder mehreren Bph-Spezies erfolgen, wobei sich die Bph an Rezeptorsubstanzen der bakteriellen äußeren Membran oder/und an Zellanhänge wie Geißeln oder Pili anheften. Besonders bevorzugt werden die Bph ausgewählt aus lambdoiden Bph, filamentösen Bphund Bph des Typs PhiX174.

Die Bph-infizierten BGs können für verschiedene Zwecke eingesetzt werden. So können in einer Ausführungsform der Erfindung Phagen verwendet werden, die ein oder mehrere heterologe Polypeptide enthalten, z. B. eingebaut in die Bph-Hülle, aber auch gegebenenfalls in ihrem Inneren. Diese heterologen Polypeptide können ausgewählt werden aus Erkennungspolypeptiden für zelluläre Rezeptoren, um eine zielgerichtete Einschleusung des Bph-modifizierten BGs in gewünschte Zelltypen, z.B. Zellen des Immensystems, zu ermöglichen. Weiterhin können die heterologen Polypeptide auch Andockpolypeptide für Enzyme, Antikörper oder andere Polypeptide, z.B. Streptavidin, sein. Auf diese Weise können weitere heterologe Polypeptide gezielt an der Außenseite der äußeren BG-Membran immobilisiert werden. Aufgrund der oftmals ortsspezifischen Anlagerung der Bph an die Bakterienoberfläche bzw. bestimmte Bakterienoberflächenstrukturen, wie z.B. äußere Membranproteine, Porine, LPS, Pili, Flagellen oder S-Layern hat diese Art der Immobilisierung Vorteile gegenüber der direkten Immobilisierung von rekombinanten Polypeptiden in der BG-Membran, die bereits aus dem Stand der Technik bekannt ist.

Noch eine weitere Anwendung ist der Bph-Display, wobei eine Population rekombinanter Phagen mit kombinatorischen Polypeptiden verwendet wird, die gewünschte DNA-Sequenzen exprimieren. Einzelne Bph aus dieser Population können dann durch Bindung der Bph-modifizierten BGs an spezifische Rezeptoren identifiziert werden.

Weiterhin können auch BGs verwendet werden, die eine heterologe Nukleinsäure enthalten, z.B. therapeutische Nukleinsäuren wie etwa Nukleinsäuren für die Gentherapie, die in Form eines chromosomal integrierbaren Vektors vorliegen können, Antisense-Nukleinsäuren, therapeutische RNA-Moleküle oder Nukleinsäure-Vakzine.

Zusätzlich oder/und alternativ kann auch der BG einen oder mehrere Wirkstoffe verpackt in seinem Inneren enthalten. Der Wirkstoff kann jeder beliebige, in das Innere der BGs transportierbare und dort vorzugsweise immobilisierbare Wirkstoff sein. Vorzugsweise wird der Wirkstoff ausgewählt aus pharmakologisch wirksamen Substanzen und Markierungssubstanzen. Beispiele für pharmakologisch wirksame Substanzen sind Polypeptide wie etwa Antikörper, therapeutisch wirksame Polypeptide wie Zytokine, Interferone, Chemokine etc., Enzyme und immunogene Polypeptide. Ein weiteres Beispiel für Wirkstoffe sind Nukleinsäuren, insbesondere therapeutische Nukleinsäuren, z.B. Nukleinsäure-Vakzine, Nukleinsäuren für die Gentherapie, die in Form eines chromosomal integrierbaren Vektors vorliegen können, Antisense-Nukleinsäuren oder Ribozyme. Noch weitere Beispiele für Wirkstoffe sind niedermolekulare Wirksubstanzen, Peptide, Hormone, Antibiotika, Antitumormittel, Steroide, Immunmodulatoren etc. Die Wirkstoffe können in den BGs in gelöster Form, als Suspensionen oder/und als Emulsionen gegebenenfalls in Kombination mit geeigneten Träger- oder/und Hilfsstoffen vorliegen. Weiterhin können die Wirkstoffe auch diagnostische Markierungssubstanzen, z.B. fluoreszierende Substanzen, Farbstoffe oder Röntgenkontrastmittel sein.

Vorzugsweise liegt der Wirkstoff in den BGs in immobilisierter Form vor, d.h. dass der verpackte Wirkstoff unter physiologischen Bedingungen eine ausreichende Zeitdauer innerhalb der BGs verbleibt, um einen Transport zur Zielzelle bzw. zum Zielgewebe zu ermöglichen. Die Immobilisierung des Wirkstoffs kann mittels kovalenter oder nichtkovalenter Wechselwirkungen, z.B. elektrostatischer Wechselwirkungen, hochaffiner biologischer Wechselwirkungen, durch mechanische Retention oder eine Kombination von zwei oder mehreren der genannten Möglichkeiten erfolgen.

Die Immobilisierung des Wirkstoffs kann über direkte oder indirekte Wechselwirkungen mit einem Rezeptor erfolgen, der auf der Membraninnenseite, z.B. der Innenseite der Zytoplasmamembran, der BGs, als integraler Membranbestandteil oder als nichtintegraler Membranbestandteil auf der Membran verankert lokalisiert ist. Der Rezeptor kann beispielsweise ein heterologes Polypeptid sein, das über einen oder mehrere Membrananker in die zytoplasmatische Membran der BGs integriert ist und durch heterologe Expression entsprechender Fusionsproteine, die mindestens eine Membranankerdomäne sowie mindestens eine Rezeptordomäne, z.B. Avidin oder Streptavidin, enthalten, in den Bakterienzellen vor der Lyse zu den BGs erzeugt wird.

Alternativ ist eine Verankerung des Rezeptors auch erst nach Lyse der BGs an der Membraninnenseite möglich, beispielsweise durch Verwendung eines Rezeptors mit zwei Bindungsstellen, wobei eine Bindungsstelle in der Lage ist, an natürliche oder rekombinante Strukturen auf der Membraninnenseite mit hoher Affinität zu binden, und die zweite Bindungsstelle für die direkte oder indirekte Immobilisierung von Wirkstoffen zur Verfügung steht.

Ein auf der Membraninnenseite der BGs lokalisiertes Rezeptormolekül kann eine direkte oder indirekte Immobilisierung von Wirkstoffen im Inneren der BGs bewirken. Bei einer direkten Immobilisierung wird ein Rezeptor gewählt, der mit dem in die BGs zu verpackenden Wirkstoff eine ausreichend starke Wechselwirkung eingehen kann, um eine weitgehende oder vollständige Retention des Wirkstoffs im BG-Inneren zu bewirken. Hierzu kann man beispielsweise mit Biotin, Haptenen oder/und Zuckern modifizierte Wirkstoffe einsetzen, die mit Rezeptoren wie Streptavidin, Antikörpern oder Lectinen eine stabile Bindung eingehen können.

Alternativ kann auch eine indirekte Immobilisierung des Wirkstoffs an den Rezeptor erfolgen, die z.B. über Wirkstoff-bindende Substanzen, die weiterhin mindestens eine zusätzliche Bindungsstelle für den Rezeptor besitzen, vermittelt wird. Beispiele für solche Wirkstoff-bindende Substanzen sind Polymere, z.B. Proteine wie Polylysin oder Polysaccharide wie etwa Protaminsulfat oder Dextran. Die Wirkstoff-bindenden Substanzen tragen weiterhin Rezeptorbindungsgruppen, z.B. Biotin oder Biotinanaloga, Haptene oder an Lectine bindefähige Zuckergruppen, die in der Lage sind, eine Verankerung an den auf der Membran lokalisierten Rezeptor zu bewirken.

Alternativ oder/und zusätzlich kann die Immobilisierung des Wirkstoffs über die Bildung einer Matrix im BG-Inneren erfolgen. Diese Matrix ist z. B. eine Polymermatrix, die im Ghostinneren in situ entsteht und das Herausdiffundieren von Wirkstoffen aus den BGs verhindert. Die Polymermatrix kann durch Polymerisation oder/und Copolymerisation geeigneter Monomere oder/und durch Zusammenlagerung aggregierbarer Substanzen im Inneren der BGs erzeugt werden. Die Polymerisation kann durch Einstellung entsprechender Bedingungen, z.B. durch Temperaturerhöhung, UV-Strahlung oder/und Zugabe geeigneter Initiatoren gestartet werden. Zweckmäßigerweise werden physiologisch verträgliche Monomere wie etwa Hydroxyfettsäuren, Aminosäuren, Saccharide oder Derivate davon verwendet, die zur Bildung eines im Körper unter physiologischen Bedingungen abbaubaren Polymeren führen.

Die Herstellung der erfindungsgemäßen mit Bakteriophagen modifizierten BGs gemäß dem zuvor genannten Aspekt der Erfindung umfasst zunächst die Herstellung der BGs nach bekannten Methoden, z.B. durch Transformation der Bakterienzelle mit einen Lysegen, vorzugsweise dem Gen E des Phagen PhiX174. Die Expression des Lysegens in der Bakterienzelle erfolgt z.B. durch eine regulierbare Expressionskontrollsequenz, wie durch das Temperatur-regulierbare Promotor/Repressor-System λ-pR/cI857. Bei diesem Expressionskontrollsystem werden die transformierten Bakterien bei Temperaturen unterhalb 30 °C kultiviert. Durch Temperaturerhöhung, z. B. auf ≥ 40 °C wird der thermosensitive λcI857 Repressor inaktiviert und das Lysegen exprimiert, was zur Ausbildung einer transmembranen Tunnelstruktur in der Zellhülle führt, wobei die Zellen innerhalb von wenigen Minuten lysiert werden. Durch mutierte λ/Promotor/Operator-Systeme ist eine Anzucht der Bakterien auch bei höheren Temperaturen, z.B. 37 °C möglich (WO98/07874). Die BGs können dann durch Zentrifugation geerntet und nach Waschen und gegebenenfalls Gefriertrocknen/Rekonstituieren mit Bph unter geeigneten Bedingungen inkubiert werden, so dass eine Adsorption der Bph an der BG-Oberfläche stattfindet. Vor oder/und nach Adsorption der Bph kann eine Beladung mit Wirkstoffen erfolgen, wobei die BGs mit einer den zu verpackenden Wirkstoff enthaltenden Lösung oder/und Suspension in Kontakt gebracht werden unter Bedingungen, die das Eindringen ausreichender Wirkstoffmengen in die BGs erlauben. Sofern erforderlich, werden darüber hinaus Rezeptorsubstanzen, die eine Immobilisierung der Wirkstoffmoleküle an der Membraninnenseite der BGs ermöglichen, oder/und matrixbildende Substanzen zugesetzt. Die Zugabe dieser Substanzen kann vor, gleichzeitig oder nach dem Inkontaktbringen der BGs mit dem zu verpackenden Wirkstoff erfolgen.

Sollte die Bph Bindung an die Bakterien eine lebende Zelle voraussetzen, so erfolgt die Infektion der Bakterien vor der Induktion der BG-Bildung durch Lyse, z.B. eine E-vermittelte Lyse. Es kann bei filamentösen Bph zudem von Vorteil sein, die Infektion der Bakterien bis zur Aufnahme der Hüllproteine der Bph in die Cytoplasmamembran ablaufen zu lassen und erst danach die E-vermittelte Lyse zu induzieren. Dies erlaubt auf natürlichen Weg das Einbringen von heterologen Proteinen in die Cytoplasmamembran von Bakterien, da diese im normalen Infektionszyklus des Bph recycelt werden und ist eine Alternative zu dem bereits patentierten Verfahren der Kompartimentierung von rekombinanten Polypeptiden in Wirtszellen (WO 00/44878).

Neben ihren Vorteilen für das Targeting bzw. den Transport von Wirkstoffen können die Bph auch zur Einstellung gewünschter physikalischer Eigenschaften, z.B. Hydrophobizität, Ladungsdichte, auf der Oberfläche von Bakterienghosts dienen. Diese Eigenschaften können durch den Bph-Beladungsgrad (Anzahl der Phagen pro Ghost) bzw. durch Modifikationen der Bph-Hüllproteine (Hydrophobizität) eingestellt werden. Zudem bestehen Bph nicht nur aus reinen Proteinstrukturen mit unterschiedlicher Größe und Gestalt sondern es gibt auch Lipid-umhüllte Bph (z.B. PM2, PRD1, Bam35 und Phi6), die damit das Spektrum der Oberflächenmodifiktion von BGs erweitern.

Die erfindungsgemäßen mit Bph modifizierten BGs können als Bestandteile pharmazeutischer Zusammensetzungen eingesetzt werden, z.B. als Vakzine oder Adjuvanzien, oder als Träger- oder/und Targetingvehikel für Wirkstoffe, wobei die Wirkstoffe im BG oder/und im Bph vorliegen können.

Die erfindungsgemäßen BGs sind hervorragend als Träger- und Targetingvehikel geeignet, da die BGs schon aufgrund ihrer Natur als Bakterienhüllen bereits bevorzugt von Zellen des Immunsystems aufgenommen werden. Verbessert werden kann dieses Targeting noch durch Verwendung von Bph-modifizierten BGs mit modifizierten Hüllen, d.h. BGs, die targetspezifische, d.h. für Zielzellen oder Zielgewebe spezifische Oberflächenmoleküle an ihrer Membranaußenseite oder/und in den Bakteriophagenhüllen tragen. Die Einführung dieser targetspezifischen Moleküle, wie etwa Antikörper, Antikörperfragmente, spezifische Liganden etc., kann durch rekombinante Expression entsprechender Fusionspolypeptide in der Bakterienzelle vor der Lyse bzw. im Bakteriophagen oder/und durch Anlagerung mittels eines geeigneten Rezeptorsystems (z.B. Streptavidin/Biotin) erfolgen.

Die Verabreichung von phagenmodifizierten Ghosts ist zur Prävention oder/und zur Bekämpfung aller Arten von Erkrankungen geeignet, z.B. zur Bekämpfung von durch Pathogene wie Viren, Bakterien, Parasiten oder Pilzen hervorgerufenen Erkrankungen, oder zur Prävention oder/und zur Bekämpfung von Tumor- oder Autoimmunerkrankungen oder zur Gentherapie. Dabei können die phagenmodifizierten Ghosts selbst oder/und darin enthaltene Wirkstoffe die erwünschte therapeutische Wirkung hervorrufen. Bei wirkstoffbeladenen Ghosts werden als Wirkstoffe gegen die jeweilige Erkrankung wirksame Substanzen verwendet, die nach Transport in der Zielzelle ihre physiologische Wirkung hervorrufen. Die vorliegende Erfindung ermöglicht auch die Verabreichung von Wirkstoffkombinationen, d.h. die BGs können mehrere verschiedene Wirkstoffe enthalten oder es können Mischungen von verschiedenen BGs gegebenenfalls mit jeweils verschiedenen Wirkstoffen verwendet werden. Weiterhin kann die Verabreichung von Wirkstoffen über BGs auch für diagnostische Zwecke (Imaging) eingesetzt werden.

Die BGs können von Gram-negativen Bakterien stammen, die z.B. ausgewählt werden aus Escherichia coli, Klebsiella, Salmonella, Pseudomonas, Vibrio, Actinobacillus, Haemophillus, Pasteurella, Bordetella und Helicobacter. Entsprechend den verwendeten Bakterienspezies erfolgt die Auswahl der Bakteriophagen wobei prinzipiell alle Gram-negativen Bakterien und ihre Bph geeignet sind

Die Verabreichung der Bph-modifizierten BGs kann nach geläufigen Methoden erfolgen, beispielsweise oral, aerogen, z.B. intranasal, intraokulär, topisch oder parenteral, z.B. intramuskulär, intraperitonial, intravenös oder subkutan.

Die Verabreichung der Ghosts kann auf demselben Weg erfolgen, wie auch eine natürliche Infektion des Organismus mit dem Erreger erfolgt. So können BGs, die zur Bekämpfung von pathogenen Erregern vorgesehen sind, deren Hautpeintrittspforte der Gastrointestinaltrakt ist (E.coli, Salmonella, Vibrio oder Helicobacter), oral verabreicht werden. Ghosts aus Erregern von Lungenentzündungen, die entsprechende Wirkstoffe enthalten, z.B. Actinobacillus, Pasteurella, Pseudomonas oder Haemophilus, können aerogen verabreicht werden.

Die Verabreichung von Bph-modifizierten und gegebenenfalls mit Wirkstoffen beladenen BGs ist nicht nur für die Humanmedizin, sondern auch für die Tiermedizin, insbesondere für die protektive Impfung von Haustieren, wie etwa Schweinen, Kühen etc., geeignet.

Die Applikation von Wirkstoffen über Bph-modifizierte BGs hat gegenüber bisherigen Applikationsformen eine Vielzahl von Vorteilen. So reichen bereits geringe Wirkstoffmengen zur Erzielung einer starken Wirkung aus. Weiterhin ist eine Zielzellen/Gewebe-spezifische Verabreichung der Wirkstoffe möglich. Aufgrund der bereits für sich immunogen wirkenden BGhüllen wird eine Adjuvanswirkung erzielt. Der in den BG eingeschlossene Wirkstoff ist vor Abbau durch physiologische Prozesse, z.B. durch Enzyme wie Proteasen, Nukleasen oder Hydrolasen geschützt. Darüber hinaus ist eine Kombination mit weiteren Wirkstoffen möglich. Schließlich können die BGs kostengünstig hergestellt und die Wirkstoffe einfach und kostengünstig formuliert werden.

Schließlich betrifft die Erfindung auch die Herstellung einer pharmazeutischen Zusammensetzung, umfassend einen Bph-modifizierten BGs. Die pharmazeutische Zusammensetzung kann in Form üblicher pharmazeutischer Präparate vorliegen, z.B. als injizierbare oder aerogen applizierbare Lösung oder Suspension, als orales Präparat, z.B. als Tablette, Kapsel oder Dragee, als Creme oder Salbe etc. Weiterhin kann die Zusammensetzung als rekonstituierbares Lyophilisat vorliegen.

Die Zusammensetzung ist erhältlich durch ein Verfahren umfassend die Schritte:
(a) Bereitstellen von Bph-infizierten Bakterien mit nachfolgender E-vermittelten BG Produktion,
(b) Bereitstellen von BGs und
(c) Inkontaktbringen der BGs mit einem Bph unter Bedingungen, die zu einer Adsorption des Bph auf in den BGs führen.

Bph stellen auf Grund ihrer Dimensionen Nanopartikel dar. BGs, die mit Bph infiziert sind, sind damit Träger von Naopartikeln. Durch Metalladsorption an Bph oder andere bereits erwähnte Modifikationen von Bph können diese so z.B. Anwendung als elektrische Leiter finden.

Weiterhin soll die vorliegende Erfindung durch das nachfolgende Beispiel erläutert werden.

### Beispiel 1

4,3 x 10⁹ *E.coli* NM522 Partikel wurden auf Weichagarplatten gegeben. Die Platten enthielten keine Bakterienghosts bzw. ein 1 mg/ml, 3 mg/ml oder 7 mg/ml Bakterienghosts (hergestellt auf bekannte Weise durch E-Lyse von E. coli NM522 Zellen).

Die in den Weichagarplatten vorliegenden Bakterienzellen wurden mit dem Phagen ϕX174 infiziert und der resultierende Phagentiter bestimmt. In Weichagarplatten ohne Zusatz von Ghosts wurde ein Phagentiter von 2 x 10¹² pfu/ml gefunden. In Platten mit 1 mg/ml Ghosts war der Phagentiter 1 x 10¹² pfu/ml. In Platten mit 3 mg/ml Ghosts war der Phagentiter 2 x 10¹⁰ pfu/ml und in Platten mit 7 mg/ml Ghosts war der Phagentiter 3 x 10⁶ pfu/ml.

Die Abnahme des Phagentiters bei Anwesenheit steigender Mengen an Bakterienghosts bedeutet, dass der Phage ϕX174 nicht nur lebende Bakterienzellen, sondern auch Bakterienghosts infiziert.

Die resultierenden Phagen-modifizierten Bakterienghosts könne auf übliche Weise, z.B. durch Zentrifugation, aus den Weichagarplatten gewonnen werden.

## Patentansprüche

1. Verfahren zur Herstellung von Bakteriophagen (Bph)-modifizierten Bakterienghosts (BG) durch Bph-Infektion von Bakterien nach der bakteriellen Lyse, umfassend
(a) Herstellen von Bakterienghosts und
(b) Inkubieren der Bakterienghosts mit Bph unter geeigneten Bedingungen, sodass eine Adsorption der Bph an der BG-Oberfläche stattfindet.

2. Verfahren nach Anspruch 1,
umfassend die weiteren Schritte
(c) Inkontaktbringen der BGs vor oder/und nach Adsorption der Bph, mit einer den zu verpackenden Wirkstoff enthaltenden Lösung oder/und Suspension unter Bedingungen, die das Eindringen ausreichender Wirkstoffmengen in die BGs erlauben,
(d) Zusetzen von Rezeptorsubstanzen, die eine Immobilisierung der Wirkstoffmoleküle an der Membraninnenseite der BGs ermöglichen, vor, gleichzeitig oder nach dem Inkontaktbringen der BGs mit dem zu verpackenden Wirkstoff, und
(e) Zusetzen von Matrix-bildenden Substanzen vor, gleichzeitig oder nach dem Inkontaktbringen der BGs mit dem zu verpackenden Wirkstoff.

3. Verfahren nach Anspruch 1 oder 2,
**dadurch gekennzeichnet,**
**dass** die Bph ausgewählt sind aus einer oder mehreren Spezies der Familien Styloviridae, Myoviridae, Podoviridae, Tectiviridae, Corticoviridae, Plasmaviridae, Microviridae, Inoviridae und Leviviridae.

4. Verfahren nach Anspruch 1 bis 3,
**dadurch gekennzeichnet,**
**dass** die Bph ausgewählt sind aus lambdoiden Bph, filamentösen Phagen, T-Phagen und Phagen des Typs PhiX174.

5. Verfahren nach Anspruch 1-4,
**dadurch gekennzeichnet,**
**dass** der Phage einen heterologen Wirkstoff, ausgewählt aus einem heterologen Polypeptid oder einer heterologen Nukleinsäure, enthält.

6. Verfahren nach Anspruch 5,
**dadurch gekennzeichnet,**
**dass** das heterologe Polypeptid ausgewählt ist aus Erkennungspolypeptiden für zelluläre Rezeptoren, Andockpolypeptiden, immunogenen Polypeptiden und kombinatorischen Polypeptiden.

7. Verfahren nach Anspruch 5,
**dadurch gekennzeichnet,**
**dass** der Phage eine heterologe Nukleinsäure, ausgewählt aus einem DNA-Vakzin, einer therapeutischen RNA oder einem Gentherapievektor, enthält.

8. Verfahren nach Anspruch 2,
**dadurch gekennzeichnet,**
**dass** der Wirkstoff ausgewählt ist aus pharmakologisch wirksamen Substanzen und Markierungssubstanzen.

9. Verfahren nach Anspruch 2 und 8,
**dadurch gekennzeichnet,**
**dass** der Wirkstoff in den BGs in immobilisierter Form vorliegt.

10. Verfahren nach Anspruch 9,
**dadurch gekennzeichnet,**
**dass** die Immobilisierung über Wechselwirkungen mit einem Rezeptor erfolgt, der auf der Membraninnenseite der BGs lokalisiert ist.

11. Verfahren nach Anspruch 9,
**dadurch gekennzeichnet,**
**dass** die Immobilisierung über die Bildung einer Matrix im BG-Inneren erfolgt.

12. Verfahren nach Anspruch 11,
**dadurch gekennzeichnet,**
**dass** die Matrix durch Polymerisation oder/und Copolymerisation von Monomeren im BG-Inneren gebildet wird.

13. Bakteriophagen (Bph)-modifizierte Bakterienghosts, erhältlich durch ein Verfahren nach den Ansprüchen 2 bis 12.

14. Verwendung der Bakteriophagen-modifizierten Bakterienghosts von Anspruch 13 für Bakteriophagen (Bph)-Display und als konstruktive Elemente in der Nanotechnologie.

15. Verfahren zur Herstellung einer pharmazeutischen Zusammensetzung, umfassend die Schritte
(a) Bereitstellen von BGs nach Anspruch 13, und
(b) Inkontaktbringen der BGs mit einem Wirkstoff unter Bedingungen, die zu einer Verpackung des Wirkstoffs in den BGs führen.

## Claims

1. Method for producing bacteriophage (bph)-modified bacterial ghosts (BG) by bph-infection of bacteria following bacterial lysis, comprising:
(a) producing bacterial ghosts and
(b) incubating the bacterial ghosts with bph under suitable conditions so that adsorption of the bph take places on the BG surface.

2. Method according to claim 1, comprising the additional steps of:
(c) bringing the BGs into contact with a solution and/or suspension containing the active ingredient to be packaged, before and/or after bph adsorption, under conditions that allow sufficient amounts of the active ingredient to enter the BGs,
(d) adding receptor substances which allow the active ingredient molecules to be immobilised on the inside of the membrane of the BGs, before, at the same time as, or after the BGs are brought into contact with the active ingredient to be packaged, and
(e) adding matrix-forming substances, before, at the same time as, or after the BGs are brought into contact with the active material to be packaged.

3. Method according to either claim 1 or claim 2, **characterised in that** the bph are selected from one or more species of the families Styloviridae, Myoviridae, Podoviridae, Tectiviridae, Corticoviridae, Plasmaviridae, Microviridae, Inoviridae and Leviviridae.

4. Method according to claims 1 to 3, **characterised in that** the bph are selected from lambdoid bph, filamentous phages, T phages and phages of the PhiX174 type.

5. Method according to claims 1 to 4, **characterised in that** the phage contains a heterologous active ingredient selected from a heterologous polypeptide or a heterologous nucleic acid.

6. Method according to claim 5, **characterised in that** the heterologous polypeptide is selected from recognition polypeptides for cell receptors, docking polypeptides, immunogenic polypeptides and combinatorial polypeptides.

7. Method according to claim 5, **characterised in that** phage contains a heterologous nucleic acid selected from a DNA vaccine, a therapeutic RNA or a gene therapy vector.

8. Method according to claim 2, **characterised in that** the active ingredient is selected from pharmacologically active ingredients and labelling substances.

9. Method according to claims 2 and 8, **characterised in that** the active ingredient is present in the BGs in immobilised form.

10. Method according to claim 9, **characterised in that** the immobilisation is achieved by interactions with a receptor which is located on the inside of the membrane of the BGs.

11. Method according to claim 9, **characterised in that** the immobilisation is achieved by the formation of a matrix in the BG interior.

12. Method according to claim 11, **characterised in that** the matrix is formed by polymerisation and/or copolymerisation of monomers in the BG interior.

13. Bacteriophage (bph)-modified bacterial ghosts, obtainable by a method according to claims 2 to 12.

14. Use of the bacteriophage-modified bacterial ghost of claim 13 for bacteriophage (bph) display and as structural elements in nanotechnology.

15. Method for producing a pharmaceutical composition, comprising the steps of:
(a) providing BGs according to claim 13, and
(b) bringing the BGs into contact with an active ingredient under conditions which lead to packaging of the active material in the BGs.

## Revendications

1. Procédé de préparation de fantômes bactériens (FB) modifiés par bactériophages (Bph), par infection par Bph de bactéries après la lyse bactérienne, comprenant :
(a) la préparation de fantômes bactériens, et
(b) l'incubation des fantômes bactériens avec un Bph dans des conditions appropriées pour qu'une adsorption du Bph sur la surface du FB se produise.

2. Procédé selon la revendication 1, comprenant les étapes supplémentaires suivantes :
(c) mise en contact des FB avant et/ou après l'adsorption du Bph, avec une solution et/ou une suspension contenant un agent actif à encapsuler, dans des conditions qui permettent l'introduction de quantités suffisantes d'agent actif dans les FB,
(d) addition de substances réceptrices, qui permettent une immobilisation de la molécule d'agent actif sur la face intérieure de la membrane du FB, avant, simultanément à ou après la mise en contact des FB avec l'agent actif à encapsuler, et
(e) addition de substances formant matrice, avant, simultanément à ou après la mise en contact des FB avec l'agent actif à encapsuler.

3. Procédé selon la revendication 1 ou 2, **caractérisé en ce que** les Bph sont choisis parmi une ou plusieurs espèces des familles des Styloviridae, des Myoviridae, des Podoviridae, des Tectiviridae, des
Corticoviridae, des Plasmaviridae, des Microviridae, des Inoviridae et des Leviviridae.

4. Procédé selon une revendication 1 à 3, **caractérisé en ce que** les Bph sont choisis parmi les Bph lambdoïdes, les phages filamenteux, les phages T et les phages de type PhiX174.

5. Procédé selon une revendication 1 - 4, **caractérisé en ce que** le phage contient un agent actif hétérologue, choisi parmi un polypeptide hétérologue ou un acide nucléique hétérologue.

6. Procédé selon la revendication 5, **caractérisé en ce que** le polypeptide hétérologue est choisi parmi les polypeptides de reconnaissance de récepteurs cellulaires, les polypeptides d'arrimage, les polypeptides immunogènes et les polypeptides combinés.

7. Procédé selon la revendication 5, **caractérisé en ce que** le phage contient un acide nucléique hétérologue, choisi parmi un vaccin à ADN, un ARN thérapeutique ou un vecteur de thérapie génique.

8. Procédé selon la revendication 2, **caractérisé en ce que** l'agent actif est choisi parmi les substances pharmacologiquement actives et les substances de marquage.

9. Procédé selon la revendication 2 et 8, **caractérisé en ce que** l'agent actif est présent dans le FB sous forme immobilisée.

10. Procédé selon la revendication 9, **caractérisé en ce que** l'immobilisation est effectuée par interaction avec un récepteur, qui se trouve sur la face intérieure de la membrane du FB.

11. procédé selon la revendication 9, **caractérisé en ce que** l'immobilisation est réalisée par formation d'une matrice à l'intérieur du FB.

12. Procédé selon la revendication 11, **caractérisé en ce que** la matrice est formée par polymérisation et/ou copolymérisation de monomères à l'intérieur du FB.

13. Fantômes bactériens modifiés par bactériophage (Bph), obtenus par un procédé selon les revendications 2 à 12.

14. Utilisation des fantômes bactériens modifiés par bactériophage selon la revendication 13, pour la présentation de bactériophage (Bph) et comme élément constructif en nanotechnologie.

15. Procédé de préparation d'une composition pharmaceutique, comprenant les étapes de :
(a) préparation de FB selon la revendication 13,
et
(b) mise en contact des FB avec un agent actif dans des conditions qui conduisent à l'encapsulation de l'agent actif dans les FB.
